(19) Europäisches Patentamt
European Patent Office
Office européen des brevets

(11) **EP 1 738 657 A2**

(12) **EUROPEAN PATENT APPLICATION**

(43) Date of publication:
**03.01.2007 Bulletin 2007/01**

(51) Int Cl.:
*A23L 1/236* (2006.01)     *A23L 1/00* (2006.01)
*A61K 47/26* (2006.01)     *A61K 9/20* (2006.01)
*A61K 9/28* (2006.01)

(21) Application number: **06013367.5**

(22) Date of filing: **28.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.06.2005 JP 2005188620**

(71) Applicant: **Kabushiki Kaisha Ueno Seiyaku Oyo Kenkyujo**
**Osaka-shi,**
**Osaka-fu (JP)**

(72) Inventors:
• **Arai, Sho**
  **Moriguchi-shi**
  **Osaka-fu (JP)**
• **Sakai, Shoko**
  **Kobe-shi**
  **Hyogo-ken (JP)**

(74) Representative: **Albrecht, Thomas et al**
**Kraus & Weisert**
**Patent- und Rechtsanwälte**
**Thomas-Wimmer-Ring 15**
**80539 München (DE)**

(54) **Sugar alcohol composition for tableting**

(57)     A powdery sugar alcohol composition which is useful as a tableting excipient is provided. The composition of the invention consists essentially of a crystalline sugar alcohol and an amorphous sugar alcohol, provided that the composition comprises 2-12 wt% of amorphous sorbitol. By employing the sugar alcohol composition as tableting excipient, pharmaceutical or confectionery tablets with enough hardness can be obtained by direct tableting under relatively low pressures.

EP 1 738 657 A2

**Description**

BACKGROUND OF THE INVENTION

FIELD OF THE INVENTION

The present invention relates to powdery sugar alcohol composition which can be used as tableting excipient suitable for manufacturing tablets by direct compression or direct tableting. The invention is also relates to pharmaceutical and confectionery tablets comprising the sugar alcohol composition as well as a process for manufacturing the same.

RELATED ART

Powdery sugar alcohols such as sorbitol and mannitol have been used as excipients for manufacturing tablets such as pharmaceutical or confectionery tablets. Particles of sugar alcohol powder, however, have only poor binding characteristics and therefore, tablets manufactured by direct tableting using said conventionally used sugar alcohol excipients may easily be broken or chipped. In order to provide tablets resistant to breakage or chipping, Japanese Patent Application Laid Open No. 2001-10979 discloses to granulate powdery sugar alcohol into granules of a predetermined size before subjecting the same to the tableting or compressing step. This is called as "granular compression" method.

Upon preparing pharmaceutical or confectionery tablets, the tableting or compressing step is carried out with relatively high pressures. However, high pressure tableting might damage the tableting press or dies and therefore, the art has desired to lower the tableting pressur to the extent the resulting tablets have enough hardness and abrasion resistance.

Conventionally used powdery sugar alcohol excipient, may cause problems like picking or sticking i.e. the powder adhere to a part of or almost whole of the surface of the tableting punch, upon tableting, especially lower pressure tableting.

Japanese Patent Laid Open No. H05-310558 discloses solid preparation composition obtained by compressing a mixture of powdery or granular sorbitol having less than 60g/100ml of bulk density and mannitol and/or lactose. The solid preparation of said patent can be obtained by direct tableting the mixture without granulating the same and the resulting tablets have good properties even if the tableting is conducted under relatively lower pressures. However, the taste of mannitol is not always preferred by consumer. In addition, the powdery or granular sorbitol used in this patent must be those specifically processed.

Upon preparing tablets, mixture of the excipient, active ingredient or food material, and additives such as lubricant, binder and degrading agent are compressed. Among the additives, lubricants are added in order to avoid attaching the mixture to the punch of the press. Since the conventionally used sugar alcohol excipients usually cause sticking or picking upon tableting, a significant amount of lubricant is always added to the conventionally used sugar alcohol excipients when they are used for manufacturing tablets by direct tableting. However, too much lubricant may deteriorate the hardness of the resulting tablets. It may also deteriorate the taste of the resulting confectionery tablets.

SUMMARY OF THE INVENTION

An object of the present invention is to provide novel powdery sugar alcohol composition which can be used as tableting excipient suitable for manufacturing tablets by direct tableting. Another object of the present invention is to provide a composition for tableting which can provide tablets containing relatively small amount of lubricants. Further object of the present invention is to provide tablets obtainable with the powdery sugar alcohol composition of the present invention and a method for manufacturing same.

Accordingly, the present invention provides a powdery sugar alcohol composition for tableting, which consists essentially of a crystalline sugar alcohol and an amorphous sugar alcohol, provided that the composition comprises 2-12 wt% of amorphous sorbitol.

The invention further provides a method for manufacturing tablets which comprises:

mixing the powdery sugar alcohol composition as above with a pharmaceutically active ingredient or a food material and a lubricant, and
tableting the mixture by direct compression.

Further more, the invention provides tablets obtained according to the method of the invention.

In the specification and claims, "crystalline sugar alcohol" represents crystallized crystallizable sugar alcohol such as maltitol. "Amorphous sugar alcohol" covers not only incrystallizable sugar alcohols but also amorphous state crystallizable sugar alcohols such as maltitol or sorbitol.

The term "direct tableting" or "tableting by direct compression" means the procedure wherein the mixture of the ingredients to be compressed into a tablet is introduced into the dies of a tablet press as a sole powdery ingredient or a powdery mixture of two or more powdery ingredients, and is then formed into tablets under pressure by means of a punch.

According to the present invention, a novel powdery sugar alcohol composition which can be used as tablet excipient suitable for manufacturing tablets by means of direct tableting is provided. By using the sugar alcohol composition of the invention, tablets with relatively high hardness containing less amount of lubricant can be manufactured by means of the direct tableting procedure, i.e. without granulating the ingredient prior to the tableting step. In addition, according to the present invention, tablets with enough hardness can be obtained by direct tableting the mixture under relatively low pressures.

DETAILED DESCRIPTION OF THE PREFERRED EMBODIMENTS

The powdery sugar alcohol composition of the present invention comprises 2-12wt%, preferably, 3-11 wt% and more preferably 4.2-10wt% of amorphous sorbitol. In the case the amount is less than 2wt%, the particles of the composition exhibit lower binding characteristics and the hardness of the resulting tablet may be decreased. In the case the amount is more than 12 wt%, flowability of the powdery sugar alcohol composition may be deteriorated and uniform dispense of the mixture to be compressed including the composition to the dies of the tablet press becomes difficult.

The crystalline sugar alcohol component contained in the powdery sugar alcohol composition of the invention may be crystals of monosaccharide- and disaccharide-alcohols. Examples may include maltitol, reduced isomaltulose, lactitol, erythritol, mannitol, xylitol and a mixture thereof.

The sugar alcohol composition of the invention may comprise one or more amorphous sugar alcohols other than amorphous sorbitol. The total amount of the amorphous sugar alcohols in the composition may be 35wt% or less, especially, 30wt% or less of the total amount of the composition. Examples of the amorphous sugar alcohols may include amorphous state maltitol, reduced isomaltulose, lactitol, erythritol, mannitol, xylitol as well as amorphous polysaccharide alcohols having three or more sugar units such as maltotriitol, and a mixture thereof. In the composition of the invention, the amount of polysaccharide alcohols is preferably less than 10 wt%.

According to the present invention, the powdery sugar alcohol composition may be obtained from starch by enzymatically decomposing the same. That is, the starch is enzymatically decomposed to give starch sugar solution, the starch sugar solution is hydrogenated to give sugar alcohol solution, and the resulting sugar alcohol solution is crystallized, dried and crushed to give powdery sugar alcohol composition. In general, in the process for manufacturing sugar alcohols from starch, enzymatically decomposed or hydrogenated syrup is purified by means of chromatography so that only disaccharides are obtained, i.e. monosaccharides, oligosaccharides as well as impurities are removed from the syrup. In the present invention, however, the powdery sugar alcohol composition prepared according to the above described enzymatic procedure but not chromatographically purified is preferably used. Sugar alcohols prepared without the chromatographic purification step contains more amorphous sorbitol than those obtained through chromatographic purification which eliminates monosaccharides and oligosaccharides from the starch sugar solution or hydrogenated starch sugar solution.

Examples of the enzymes used in the enzymatic decomposition of starch may include commercially available amylases such as $\alpha$-amylase, $\beta$-amylase and pullulanase. Those enzymes may be used as a sole enzyme or in combination of two or more. The enzymatic decomposition may be conducted as a two or more steps process.

The sugar solution obtained by the enzymatic decomposition of starch is then hydrogenated by processing the solution under the presence of hydrogen and a catalyst such as Raney nickel at high temperatures and pressures to give sugar alcohol solution. The sugar solution obtained by the enzymatic decomposition of starch may preferably be purified with ion exchange resin and/or activated carbon before being hydrogenated.

Thus obtained sugar alcohol solution may be concentrated, crystallized and then, crushed to give powdery sugar alcohol composition. The sugar alcohol solution obtained by hydrogenation of the sugar solution may preferably be purified with ion exchange resin and/or activated carbon before being concentrated.

The powdery sugar alcohol composition of the present invention, which is preferably used as tableting excipient, may comprise 88-98wt% of maltitol and 2-12wt%, especially 4.2-10wt% of amorphous sorbitol. The amount of maltitol is more preferably 88-95.8wt%, especially, 89-92wt%. The maltitol component in the composition may contain amorphous maltitol so long as the total amount of amorphous sugar alcohols in the composition is 35wt% or less, preferably, 30wt% or less.

The particle size of the powdery sugar alcohol composition of the present invention is not limited and may be adjusted by sifting the particles depending on the desired property of the resulting tablets. For example, in the case of pharmaceutical or confectionery tablets with high hardness are preferable, particle size of the powdery composition may preferably be within the range of about 1-295$\mu$m.

According to the present invention, pharmaceutical or confectionary tablets may be manufactured by mixing the powdery sugar alcohol composition of the present invention, a lubricant and a pharmaceutically active ingredient or food ingredient, and direct tableting the mixture.

According to the present invention, the amount of the powdery sugar alcohol composition is not limited and may be adjusted based on the desired property of the resulting tablets. The amount may be 20-99wt%, preferably 50-99wt% of total amount of the mixture.

Lubricants which may be used for manufacturing the tablet of the present invention are not limited and any of those designated as food additives may be used. Examples of lubricants may include sucrose fatty acid ester, calcium stearate, magnesium stearate and glycerine fatty acid ester. Among them, sucrose fatty acid ester, calcium stearate and magnesium stearate are preferable in view of their good lubricating property. The amount of lubricant in the mixture is not limited and may be adjusted based on the desired property of the resulting tablets. For example, the amount of the lubricant may be 10wt% or less, more preferably, 0.1-5wt% of the mixture.

According to the present invention, the confectionery tablets are not limited and may be those for providing refreshing or pleasant flavor of, for example, mint or fruits, for preventing bad breath or cavities, for soothing throat, and the like. The tablets may be those to be chewed or sucked.

According to the present invention, the food ingredients are not specifically limited and those conventionally incorporated in tablets may be employed. Examples of food ingredients may include dried bean paste, powdered tea, powdered fruit juice and dried fruits.

The pharmaceutically active ingredient used in the present invention is not limited and any of those conventionally provided in the form of tablets may be employed. In the tablet of the present invention, the pharmaceutically active ingredient may be comprised as a sole agent or in combination of two or more agents.

The amount of the pharmaceutically active ingredient may vary depending on the type of the agent and age, body weight, symptom to be treated, desired therapeutic effect, term of treatment and the like.

According to the present invention, the mixture to be tabletted or compressed may further comprise one or more additives. The additives used for preparing tablets of the invention are not limited and any of those used in manufacturing conventional tablets can be employed. Examples of the additives may include degrading agents such as crystalline cellulose, crystalline cellulose derivatives and chemically-modified starch, binders such as alpha starch and pullulan, excipients other than sugar alcohols such as lactose, sucrose and glucose, flavor, colorant and sour agent. The amount of the additives may be determined based on the desired property of the resulting tablets.

The total amount of the ingredients other than the sugar alcohol composition of the invention and lubricant in the mixture to be tabletted may be 79.9wt% or less, and preferably, 0.1-50 wt%.

As is discussed above, the mixture is not necessarily granulated prior to being compressed. However, if desired, the mixture may be granulated before compression.

The tableting or compressing of the mixture may be carried out using a conventional eccentric or rotary type tableting press. By employing the powdery sugar alcohol composition of the invention as a tableting excipient, tablets can be manufactured with relatively low pressures.

EXAMPLES

The instant invention is further illustrated by the following examples.

EXAMPLE 1

Preparation of powdery sugar alcohol composition:

Tapioca starch slurry in water was added with α-amylase to decompose and liquefy the starch. The resulting liquid was cooled and added with β-amylase and pullulanase to saccharify the starch. After that, the α-amylase was added to the mixture to further proceed the saccharification. The resulting sugar solution was filtrated through activated carbon filter and then demineralized with ion-exchange resin. Thus purified sugar solution was concentrated, and hydrogenated under the presence of hydrogen and Raney nickel at a temperature of 90-140°C and a pressure of 20-100 bar to give aqueous sugar alcohol solution. Thus obtained sugar alcohol solution was purified with activated carbon filter and ion-exchange resin, concentrated, crystallized, crushed and dried to give powdery sugar alcohol composition of example 1. The components in the composition are shown in table 1 below. The particle size of the powdery composition and the amount of the amorphous sorbitol in the composition were determined by the procedure shown below.

As a sugar alcohol composition of comparative example 1, AMALTY MR50 (TOWA Chemical Industries, Co. Ltd., Tokyo, Japan) was used. The components of AMALTY MR 50 are also shown in Table 1.

Measurement of Particle Size

Particle size of the composition was determined by means of dry particle size measuring method using JIS sieves.

In the measurement, JIS sieves having opening sizes of 106μm, 149μm, 180μm, 210μm, 250μm and 300μm were used. Fifty grams of the sample powder was put on the respective sieve and shaken for 10 minutes at 25°C and 50%RH. The amount of the sample remained on the sieve was measured. The particle size distribution was determined by means of Rosin-Rammler plot, and the average particle size was obtained from the particle size distribution.

Amount of the amorphous sorbitol

The sample was dried in vacuum desiccator at room temperature for 1 hour and approximately 10mg of the dried sample was put in an aluminum pan. The sample was scanned with differential scanning calorimeter (DSC 6200: Seiko Instruments Inc., Chiba, Japan) over a temperature range of 30-180°C at a rate of 4°C/min to determine latent heat of melting of the obtained crystal over 60-100°C. The obtained latent heat of melting was assumed attributable to crystalline sorbitol in the sample and the amount of the crystalline sorbitol in the sample was calculated based on the formula as follows:

$$Amount\ of\ crystalline\ sorbitol\ in\ the\ sample(wt\%\ on\ DS*)=$$

$$\frac{measured\ latent\ heat\ of\ melting}{latent\ heat\ of\ melting\ of\ pure\ crystalline\ sorbitol(200)} \times 100$$

*DS: Dry Substance

$$Amount\ of\ amorphous\ sorbitol\ in\ the\ sample(wt\%\ on\ DS)=$$

$$(amount\ of\ total\ sorbitol\ in\ the\ sample(wt\%\ on\ DS))-(amount$$

$$of\ crystalline\ sorbitol\ in\ the\ sample(wt\%\ on\ DS))$$

Amount of the amorphous maltitol
The sample was dried in vacuum desiccator at room temperature for 1 hour and approximately 10mg of the dried sample was put in an aluminum pan. The sample was scanned with differential scanning calorimeter (DSC 6200: Seiko Instruments Inc., Chiba, Japan) over a temperature range of 30-180°C at a rate of 4°C/min to determine latent heat of melting of the obtained crystal over 120-160°C. The obtained latent heat of melting was assumed attributable to crystalline maltitol in the sample and the amount of the crystalline maltitol in the sample was calculated based on the formula as follows:

$$Amount\ of\ crystalline\ maltitol\ in\ the\ sample(wt\%\ on\ DS)=$$

$$\frac{measured\ latent\ heat\ of\ melting}{latent\ heat\ of\ melting\ of\ pure\ crystalline\ maltitol(165)} \times 100$$

$$\text{Amount of amorphous maltitol in the sample(wt\% on DS)=}$$

$$\text{(amount of total maltitol in the sample(wt\% on DS))-(amount}$$

$$\text{of crystalline maltitol in the sample(wt\% on DS))}$$

The amount of crystal and non-crystal in the sample were determined as follows:

$$\text{Amount of crystal in the sample (wt\% on DS)=}$$

$$\text{(amount of crystalline sorbitol (wt\% on DS)) + (amount of}$$

$$\text{crystalline maltitol(wt\% on DS))}$$

$$\text{Amount of non-crystal in the sample (wt\% on DS)=}$$

$$\text{100-(amount of crystal in the sample(wt\% on DS))}$$

Table 1: Sugar alcohol compositions

|  | sugar alcohol content wt% on DS | | | weight ratio of crystal: non-crystal in the sample | amorphous sorbitol wt% on DS | particle size range $\mu$m | average particle size $\mu$m |
|---|---|---|---|---|---|---|---|
|  | maltitol | sorbitol | other sugar alcohols |  |  |  |  |
| Ex.1 | 90.0 | 4.7 | 5.3 | 72:28 | 4.7 | 1-295 | 140 |
| com. Ex. 1 | 94.5 | 1.0 | 4.5 | 75:25 | 1.0 | 1-295 | 140 |
| Tableting (or compressing) | | | | | | | |

The above obtained powdery sugar alcohol composition for tableting of the example 1 or comparative example 1 was admixed with 0.5wt% of magnesium stearate (lubricant, Taihei Chemical Industrial Co., Ltd.). Tablets were formed by direct tableting under the condition shown below using rotary tablet press (AQU3 0512SW2AIII, Kikusui Seisakusho, Ltd.). The mixture comprising the powdery sugar alcohol composition for tableting of the present invention did not cause any problem such as picking or sticking upon the tableting procedure. The hardness of the resulting tablets was determined with Kiya hardness tester (Fujiwara Scientific Company Co., Ltd) and confirmed the tablet of the invention had a relatively high hardness. Results are shown in Table 2.

In comparative example 1, the mixture comprising the composition was attached to a part of the punch of the tablet press, i.e. picking was occurred, and tableting could not be completed.

Tableting Condition:
| | |
|---|---|
| Die: | $\varphi$8mm, R12mm |
| Tablet weight (per one tablet): | 250mg |
| Rotation speed of the rotary station: | 10rpm |
| Tableting pressure: | 0.5 t |

Table 2

| Example 1 | tablettability | Yes |
|---|---|---|
| | hardness (kg) | 12.9 |
| Com. Ex. 1 | tablettability | No |
| | hardness (kg) | - |
| Tablettability<br>Yes: complete tablets were obtained<br>No: complete tablets were not obtained<br>-: No data | | |

Example 2

The powdery sugar alcohol composition for tableting of Example 1 as shown in Table 1 was admixed with 2 wt%, 3 wt%, 5wt% and 7 wt% of a sucrose fatty acid ester lubricant (DK ester H-5423, Dai-ichi Kogyo Seiyaku Co., Ltd., Kyoto, Japan) to give mixtures. The respective mixture was then subjected to direct tableting with eccentric tablet press (Hayakawa Seisakusyo, Japan) under the condition shown below. All mixtures did not cause any problem such as picking or sticking and could provide complete tablets. Results are shown in Table 3.

As comparative example 2, the sugar alcohol composition of comparative example 1 was used and admixed with the same amount of the lubricant as example 2. In the comparative example 2, regardless of the amount of the lubricant, the powdery mixture was attached to the surface of the punch and only deficient tablets were obtained. Results are shown in Table 3.

Tableting Condition

| | |
|---|---|
| Die: | φ8mm, R 6. 5mm |
| Tablet weight (per one tablet): | 300mg |
| Tableting pressure: | Normal pressure |

Table 3

| Tablettability | | | | |
|---|---|---|---|---|
| | amount of the lubricant added | | | |
| | 2% | 3% | 5% | 7% |
| Example 2 | Yes | Yes | Yes | Yes |
| Com. Ex. 2 | No | No | No | No |
| Tablettability:<br>Yes: complete tablets were obtained<br>No: complete tablets were not obtained due to sticking<br>-: No data | | | | |

Examples 3 and 4

The sugar alcohol composition for tableting of example 1 as shown in Table 1 was admixed with a sucrose fatty acid ester lubricant (DK-ester F-20W, Dai-ichi Kogyo Seiyaku Co., Ltd., Kyoto, Japan) in an amount of 5wt% and 8wt% to give mixtures of examples 3 and 4 respectively. As comparative examples 3 and 4, mixtures comprising the sugar alcohol composition of comparative example 1 and the sucrose fatty acid ester lubricant used in Examples 3 and 4 in an amount of 5wt% and 8wt% were used respectively.

The mixtures of examples and comparative examples were subjected to direct tableting under the condition shown below using the rotary tablet press (AQU3 0512SW2AIII, Kikusui Seisakusho, Ltd.) at tableting pressures of 0.3, 0.5 and 0.8 tons.

When the tableting pressure was 0.5t or more, the mixtures comprising the sugar alcohol composition of Examples 3 or 4 did not cause any problem such as picking or sticking and could provide complete tablets. The hardness of the resulting tablets was determined with Kiya hardness tester (Fujiwara Scientific Company Co., Ltd) and confirmed that the hardness of the tablets of examples 3 and 4 were higher than that of comparative example 4.

In comparative examples, the mixture containing 5wt% of the lubricant were attached to the surface of the punch and caused picking or sticking upon tableting, and could provide only deficient tablets. Mixtures those containing 8wt% of the lubricant could provide complete tablets when tabletted with a pressure of 0.5t or more. However, the hardness of the resulting tablets were lower than those obtained by the invention. Results are shown in Tables 4 and 5.

Tableting Condition:

| | |
|---|---|
| Die: | φ8mm, R12mm |
| Tablet weight(per one tablet): | 250mg |
| Rotation speed of the rotary station: | 10rpm |
| Tableting pressure: | 0.3t, 0.5t and 0.8t |

Table 4

| The mixture containing 5wt% of lubricant | | | | |
|---|---|---|---|---|
| | | tableting pressure (t) | | |
| | | 0.3 | 0.5 | 0.8 |
| Ex. 3 | tablettability | No | Yes | Yes |
| | hardness(kg) | - | 11.5 | 20.4 |
| Comp. Ex. 3 | tablettability | No | No | No |
| | hardness(kg) | - | - | - |
| Tablettability<br>Yes: complete tablets were obtained<br>No: complete tablets were not obtained due to sticking<br>-: No data | | | | |

Table 5

| The mixture containing 8wt% of lubricant | | | | |
|---|---|---|---|---|
| | | tableting pressure (t) | | |
| | | 0.3 | 0.5 | 0.8 |
| Ex. 4 | tablettability | Yes | Yes | Yes |
| | hardness(kg) | 6.0 | 9.3 | 18.5 |
| Comp. Ex. 4 | tablettability | No | Yes | Yes |
| | hardness (kg) | - | 8.3 | 14.3 |
| Tablettability<br>Yes: complete tablets were obtained<br>No: complete tablets were not obtained due to sticking<br>-: No data | | | | |

Examples 5-7

A powdery sugar alcohol composition for tableting of Example 5 shown in Table 6 was obtained according to the same manner as example 1. Powdery sugar alcohol compositions for tableting of Examples 6 and 7 as well as comparative example 5 shown in Table 6 were obtained by dissolving crystalline maltitol (Lesys®, Towa Chemical Industry Co., Ltd., Tokyo, Japan) and D-sorbitol (Merck Japan, Tokyo, Japan in water, concentrating the solution, crystallizing the concentrated syrup, crushing the crystallized product and drying the same.

Further, sugar alcohol composition of comparative examples 6-8 shown in table 6 were obtained by mixing crushed crystalline maltitol (Lesys®, Towa Chemical Industry Co., Ltd., Tokyo, Japan) and crushed crystalline sorbitol (Neosorb® P60W, ROQUETTE Japan, Tokyo, Japan). Those crushed sugar alcohols were sieved on a 60M sieve and mixed so that the amount of maltitol and sorbitol in the composition were those shown in Table 6.

Particle Size

Particle size of the powdery sugar alcohol composition was determined with Mastersizer 2000E (Malvern Instruments Ltd, UK) according to the wet particle size measurement method using isopropanol as solvent. The average particle size was shown as mean volume diameter.

Amount of amorphous sorbitol and weight ratio of crystal:non-crystal in the sample were determined according to the same manner as Example 1.

Results are shown in Table 6 below:

Table 6: Sugar alcohol compositions

|  | sugar alcohol content wt% on DS | | | weight ratio of crystal: non-crystal in the sample | amorphous sorbitol wt% on DS | particle size range μm | average particle size μm |
|---|---|---|---|---|---|---|---|
|  | maltitol | sorbitol | other sugar alcohols |  |  |  |  |
| Ex. 5 | 90.7 | 4.5 | 4.8 | 72.7:27.3 | 3.8 | 1-399 | 125.9 |
| Ex. 6 | 88.0 | 10.5 | 1.5 | 79.5:20.5 | 6.6 | 1-317 | 81.4 |
| Ex. 7 | 94.2 | 4.6 | 1.1 | 87.7:12.3 | 2.8 | 1-356 | 74.6 |
| Comp. Ex. 5 | 99.2 | 0.1 | 0.7 | 99.2: 0.8 | 0.0 | 1-252 | 58.8 |
| Comp. Ex. 6 | 90.1 | 9.5 | 0.4 | 90.8: 9.2 | 0.0 | 1-317 | 76.9 |
| Comp. Ex. 7 | 95.2 | 4.5 | 0.4 | 95.7: 4.3 | 0.0 | 1-317 | 76.5 |
| Comp. Ex. 8 | 99.7 | 0.0 | 0.3 | 99.7: 0.3 | 0.0 | 1-356 | 78.4 |
| Tableting (or compressing) | | | | | | | |

The above obtained powdery sugar alcohol composition for tableting of examples 5-7 and comparative examples 5-8 were admixed with 2wt% of a sucrose fatty acid ester lubricant (DK-ester F-20W, Dai-ichi Kogyo Seiyaku Co., Ltd., Kyoto, Japan) and 28wt% of dried bean paste (*KAORI* FR, Ueno Fine Chemicals Industry Ltd., Osaka, Japan). Tablets were formed by direct tableting under the condition shown below using Piccola B-10 rotary tablet press (Riva). The hardness of the resulting tablets was determined with Kiya hardness tester (Fujiwara Scientific Company Co., Ltd). Tablets obtained from the mixture comprising the powdery sugar alcohol composition of the examples exhibited higher hardness than those obtained from comparative examples. Results are shown in Table 7.

Tableting Condition:
Die:                                    φ8mm, R12mm
Tablet weight(per one tablet):          300mg
Rotation speed of the rotary station:   10rpm
Tableting pressure:                     0.5t and 1.0t

Table 7: Hardness of tablets

|  | hardness(kg) | |
|---|---|---|
|  | (0.5t) | (1.0t) |
| Example5 | 8.0 | 11.3 |

(continued)

|  | hardness(kg) | |
|---|---|---|
|  | (0.5t) | (1.0t) |
| Example6 | 5.7 | 8.6 |
| Example7 | 4.1 | 7.3 |
| Comp. Ex.5 | -* | 6.2 |
| Comp. Ex.6 | 1.6 | 2.4 |
| Comp. Ex.7 | 1.8 | 2.9 |
| Comp. Ex.8 | 2.1 | 4.1 |
| * complete tablets were not obtained | | |

## Claims

1. A powdery sugar alcohol composition for tableting, which consists essentially of a crystalline sugar alcohol and an amorphous sugar alcohol, provided that the composition comprises 2-12 wt% of amorphous sorbitol.

2. The composition of Claim 1, wherein the amount of the amorphous sorbitol contained in the composition is 4.2-10 wt%.

3. The composition of Claim 1 or 2, wherein the composition comprises 88-98wt% of maltitol.

4. The powdery sugar alcohol composition, which is obtained by a method comprising the steps of: enzymatically decomposing starch to give starch sugar solution, and hydrogenating the starch sugar solution to give sugar alcohol solution, wherein the starch sugar solution as well as sugar alcohol solution are not subjected to chromatograph purification.

5. The composition of any of Claims 1-4, wherein the amount of total amorphous sugar alcohols is 35wt% or less.

6. The composition according to any of Claims 1-5, wherein the particle size of the powdery sugar alcohol composition within the range of 1-295μm.

7. A method for manufacturing tablets which comprises the steps of:

   mixing the powdery sugar alcohol composition according to any of claims 1-6, a lubricant and a food ingredient or a pharmaceutically active ingredient;
   tableting the mixture.

8. The method according to claim 7, wherein the mixture comprises 20-99wt% of the powdery sugar alcohol composition and 0.1-5wt% of lubricant.

9. The method according to claim 7 or 8, wherein the tableting is conducted by direct tableting.

10. Tabletted product obtained by tableting a mixture comprising the sugar alcohol composition of any one of claims 1-6, a lubricant and a food ingredient or a pharmaceutically active ingredient.

11. The product of Claim 10, which is manufactured by direct tableting.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001010979 A
- JP H05310558 A